# EUROPEAN PATENT APPLICATION

(11) **EP 2 371 351 A1**
(43) Date of publication of application: **05.10.2011**
(21) Application number: 10003676.3
(22) Date of filing: 01.04.2010
(51) Int. Cl.: A61K 9/00, A61K 31/167, A61P 17/02

(54) **Pharmaceutical composition for the treatment of solar urticaria**

(71) Applicant: Charité Universitätsmedizin Berlin, 10117 Berlin (DE)
(72) Inventor: Maurer, Marcus, 14055 Berlin (DE); Metz, Martin, 14169 Berlin (DE); Krause, Karoline, 10119 Berlin (DE)
(74) Representative: Engelhard, Markus

(57) **Abstract**

The present invention refers to a local anesthetic, preferably selected from the group of aminoamide local anesthetics, in particular lidocaine or prilocaine, for use in the treatment or prevention of physical urticaria, in particular solar urticaria. The present invention further refers to a pharmaceutical composition, e.g. a cream, comprising such a local anesthetic, in particular a combination of lidocaine and prilocaine.

## Description

The present invention refers to local anesthetics, in particular lidocaine and/or prilocaine, in the treatment or prevention of physical urticaria, in particular solar urticaria.

### Background of the invention

Physical urticarias are characterized by wheal-and-flare-type skin reactions and/or angioedema following exposure to a specific, exogenous, physical stimulus. The different subtypes are classified according to their eliciting physical trigger. These include thermal triggers (cold or heat contact), electromagnetic radiation (usually UVA or visible light), and mechanical triggers (friction, pressure, or vibration) (Zuberbier *et al.*, 2006).

The symptoms of physical urticarias are caused by the activation of skin mast cells and subsequent release of histamine and other pro-inflammatory mediators including cytokines. As a result patients experience pruritus and/or burning sensations. Skin blood vessels show signs of dilation and increased extravasation, which allows erythema and edema (wheal or angioedema) to form. However, it is largely unknown how and why mast cells are activated in patients with physical urticaria, i.e. what signals are responsible for mast cell activation and what causes them to come about.

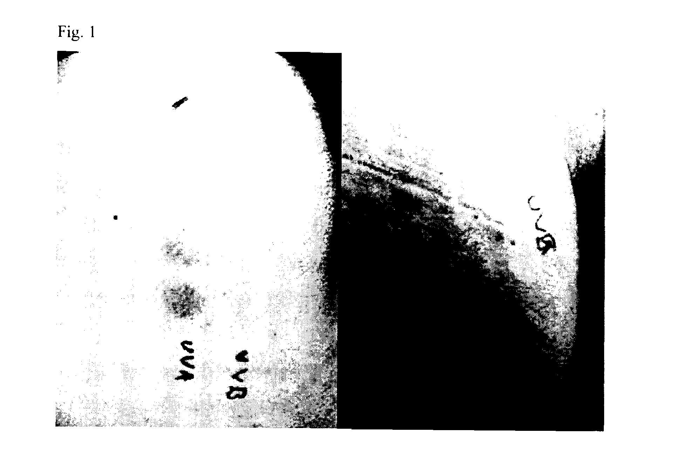

Solar urticaria is characterized by the early onset of itching, erythema, and whealing after exposure to UVA, visible light, less commonly UVB and rarely infrared radiation. Typically, symptoms occur within minutes after solar radiation and are limited to sun-exposed skin areas (Figure 1).

In contrast to physical urticaria, the pathogenesis of allergic urticaria is well known. There, the trigger is the allergen that causes an allergic response. By binding of the antigen to mast cells, degranulation is initiated and causes release of histamine.

The pathogenesis of solar urticaria, however, is still poorly understood. Because solar urticaria symptoms are easy to induce, it is a suitable model condition to assess triggers, mechanisms and causes of physical urticaria. One of the favoured hypothesis is that irradiation with light produces a photoallergen within the skin which crosslinks specific immunoglobulin E (IgE) bound to the FcεRI-receptor of cutaneous mast cells. The ensuing mast-cell degranulation then leads to the release of a wide range of mediators, including histamine, which cause pruritus and the typical wheal and flare reaction. Yet, the hypothesis is not established.

Nonsedating H1-antihistamines are the first line therapy for solar urticaria, but in severe cases conventional antihistaminic treatment is often unsuccessful. High-grade sun blockers are usually not sufficient, and light-hardening therapy, although sometimes quite effective, is not appropriate for long-term treatment because of the risk of skin tumour development.

Anti-IgE therapy (omalizumab) has been shown to be effective in a single case of severe antihistamine-resistant solar urticaria (Güzelbey *et al.*, 2008). However, omalizumab is currently not licensed for urticaria, is very expensive, and data from clinical trials with larger patient numbers are missing.

US 2008/0221206 discloses lidocaine derived compounds and pharmaceutical compositions for use in the treatment of allergic urticaria, which is a different type of urticaria underlying a different mode of triggering urticaria-like symptoms.

Accordingly, the object to be solved is to provide methods and means for an effective treatment of physical urticaria, in particular solar urticaria.

### Summary of the invention

The object of the present invention is solved by a local anesthetic for use in the treatment or prevention of physical urticaria in a subject.

In one embodiment, the local anesthetic is selected from the group of aminoamide local anesthetics.

In one embodiment, the local anesthetic is lidocaine, or a derivative thereof, or is prilocaine, or a derivative thereof.

The object of the present invention is also solved by a combination of lidocaine, or a derivative thereof, and prilocaine, or a derivative thereof, for use in the treatment or prevention of physical urticaria in a subject.

The object of the present invention is further solved by a pharmaceutical composition comprising at least one local anesthetic for use in the treatment or prevention of physical urticaria in a subject. One, two or more different local anesthetics are considered.

In one embodiment of the pharmaceutical composition, the at least one local anesthetic is one selected from the group of aminoamide local anesthetics.

In one embodiment of the pharmaceutical composition, the at least one local anesthetic is lidocaine, or a derivative thereof, or is prilocaine, or a derivative thereof.

In one embodiment, the pharmaceutical composition comprises both lidocaine, or a derivative thereof, and prilocaine, or a derivative thereof.

In one embodiment, the pharmaceutical composition comprises both lidocaine, or a derivative thereof, and prilocaine, or a derivative thereof, and, in addition, at least one further local anesthetic, preferably selected from the group of aminoamide local anesthetics.

In one embodiment, the pharmaceutical composition further comprises additives such as a sun protection factor or skin care agents.

In one embodiment, the pharmaceutical composition is formulated to be administered non-systemically, e.g. locally, topically, transdermally, or subcutaneously.

In one embodiment, the pharmaceutical composition is comprised by or formulated as a pharmaceutical dosage form selected from the group consisting of a cream, an ointment, a salve, an elixier, a lotion, a solution, a patch, an emulsion, a foam, and a spray. Such dosage forms may be applied by spreading, spraying, or by injection. Controlled-release dosage forms, e.g. a patch for sustained-release, are also considered. Dosage forms prepared to be administered in an individual dosage regimen are also considered.

In one embodiment, the pharmaceutical composition is comprised by or formulated as a cream, preferably a skin cream, comprising 2.5% by weight lidocaine and 2.5% by weight prilocaine.

In a preferred embodiment, the pharmaceutical composition is EMLA^{®} cream.

In one embodiment, the cream is used at a dosage of 5 g per site.

In one embodiment, the pharmaceutical composition is comprised by or formulated as a patch comprising 25 mg lidocaine and 25 mg prilocaine per patch.

In a preferred embodiment, the pharmaceutical composition is comprised by EMLA^{®} patch.

The object of the present invention is further solved by a use of at least on local anesthetic, preferably lidocaine and/or prilocaine, for the preparation of a pharmaceutical composition for the treatment or prevention of physical urticaria in a subject.

The object of the present invention is further solved by a use of a combination of lidocaine, or a derivative thereof, and prilocaine, or a derivative thereof, for the preparation of a pharmaceutical composition for the treatment or prevention of physical urticaria in a subject.

The object of the present invention is further solved by a method of using at least one local anesthetic, preferably selected from the group of aminoamide local anesthetics, most preferably lidocaine and/or prilocaine, for the treatment or prevention of physical urticaria in a subject.

The object of the present invention is further solved by a method of using a combination of lidocaine, or a derivative thereof, and prilocaine, or a derivatiave thereof, for the treatment or prevention of physical urticaria in a subject.

In one embodiment of the present invention, i.e. in one embodiment of the local anesthetic, the combination, the pharmaceutical composition, the use, and the method, the physical urticaria is selected from the group consisting of cold contact urticaria, delayed pressure urticaria, heat contact urticaria, urticaria factitia (also referred to as dermographic urticaria), vibratory urticaria (angioedema), and solar urticaria.

In a particularly preferred embodiment of the present invention, the physical urticaria is solar urticaria.

In one embodiment of the present invention, the subject is a subject in need of a treatment or prevention of physical urticaria, preferably solar urticaria. Preferably, the subject is a mammal, most preferably a human. Particularly preferred is a subject having solar urticaria and being sensitive to UVA-radiation, i.e. responding to UVA-radiation by developing symptoms of solar urticaria.

The term "a local anesthetic" refers to a drug that causes reversible local anesthesia and a loss of nociception. Chemically, the group of local anesthetics comprises aminoesters ("ester type") and aminoamides ("amide type"). Local anesthetics may be used for topical anesthesia, infiltration, plexus block, epidural block, and spinal anesthesia. For use in the present invention, aminoamide local anesthetics, aminoester local anesthetics and other types of local anesthetics are considered.

The group of "aminoamide local anesthetics" comprises lidocaine (lignocaine, xylocaine), prilocaine, articaine, bupivacaine, cinchocaine (dibucaine), etidocaine, levobupivacaine, mepivacaine, piperocaine, ropivacaine, and trimecaine.

A "physical urticaria" may be also referred to as "non-allergic urticaria" in order to distinguish this type of urticaria from urticaria of allergic pathogenesis.

For use "in the treatment" of physical urticaria, preferably solar urticaria, means a use in a subject showing symptoms of the disease. In this case, skin areas showing the symptoms may be treated.

For use "in the prevention" of physical urticaria, preferably solar urticaria, means a use in a subject without established symptoms of the disease. The subject may be one in whom the disease has been diagnosed previously, or may be one being at risk of developing the disease, e.g. due to a familiar disposition. In this case, skin areas to be exposed to e.g. sun light may be treated.

The term "a derivative", e.g. a derivative of lidocaine, refers to a compound chemically derived from lidocaine or being related in chemical structure. Similarly, a derivative of prilocaine refers to a compound chemically derived from prilocaine or being related in chemical structure. Pharmaceutically acceptable salts of lidocaine or prilocaine or their derivatives are also considered.

The term "a combination" of lidocaine, or a derivative thereof, and prilocaine, or a derivative thereof, means that the active compounds are used in conjunction. Thus, the active compounds may be comprised by one and the same pharmaceutical composition. Alternatively, each active compound may be comprised singly by a pharmaceutical composition, and the pharmaceutical compositions are used together.

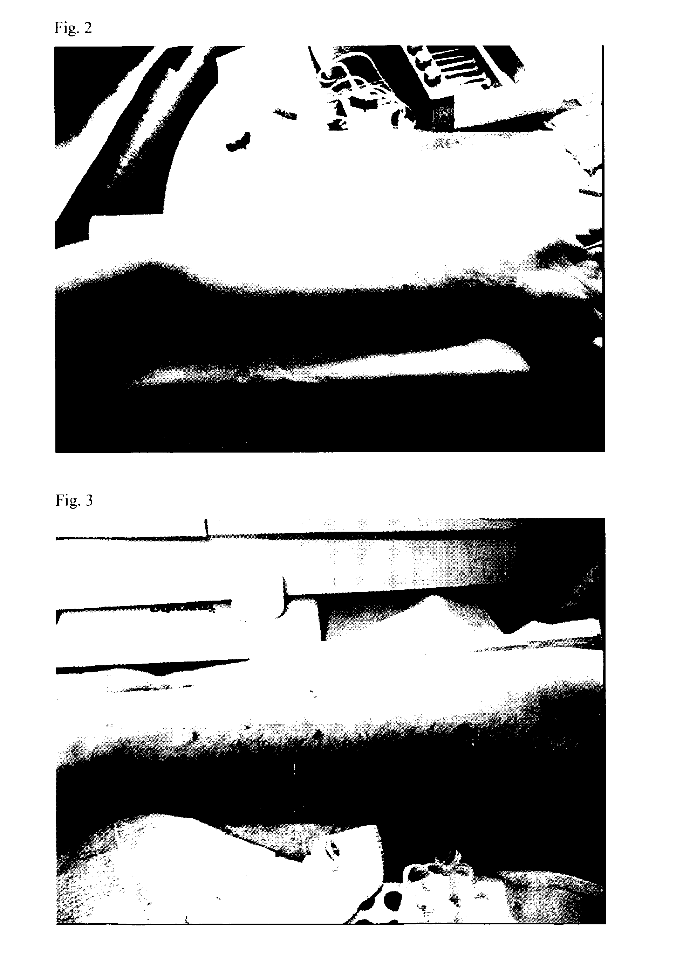

The aim of the study underlying the present invention was to investigate pathogenic mechanisms in a type of physical urticaria, i.e. solar urticaria. A pharmaceutical composition (EMLA^{®} cream) comprising lidocaine and prilocaine was used as a topical anesthetic on skin parts of patients to be exposed to UV-radiation. Surprisingly, the topical application of the local anaesthetic EMLA^{®} cream resulted in a reduction of clinical symptoms of solar urticaria (wheals, erythema, pruritus; Figure 3).

This effect was further demonstrated by comparing parts of the skin exposed to UV-radiation with and without prior application of EMLA^{®} (Figures 4 and 5). These findings were confirmed by digital time lapse photography (Figure 6).

The effectiveness of EMLA^{®} suggests that an application of the active compounds comprised in EMLA^{®} prior to UV-provocation stabilizes or inhibits sensory nerve fibres of the skin and induces down-regulation or inhibition of the release of pro-inflammatory neuropeptides. Due to this more general concept it is assumed that (1) other local anesthetics in addition to lidocaine and prilocaine, (2) lidocaine alone or prilocaine alone, and (3) lidocaine and/or prilocaine in combination with other local anesthetics are effective as well.

In summary, it was demonstrated in two of two patients with solar urticaria and an action spectrum to UVA that the combination of lidocaine and prilocaine protects the tissue and inhibts triggering of the cascade which usually leads to typical symptoms of this disease. These results suggest the use of EMLA^{®} as a treatment option in solar urticaria patients. Such a treatment is not only effective, as demonstrated herein, but is also convenient to the patient since a pharmaceutical composition may be topically applied and, moreover, is associated with low side-effects.

### Detailed description of the invention

The present invention shall now be described in more detail on the basis of the following examples and with reference to the attached figures.
Figure 1 shows symptoms of solar urticaria on the skin of the buttock after UVA- and UVB provocation. In this case, both patients were sensitive to UVA-radiation, but not to UVB-radiation. The skin shows erythema and wheals where exposed to UVA-radiation.
Figure 2 shows a volar forearm of a patient 1.5 hours after EMLA^{®} application. The skin shows pallor where the anesthetic cream has been applied due to vascoconstriction.
Figure 3 shows negative reaction to UVA-provocation in EMLA-treated skin after 20 min on volar forearm of a patient during microdialysis. The duration of UVA-provocation was 3.35 min.

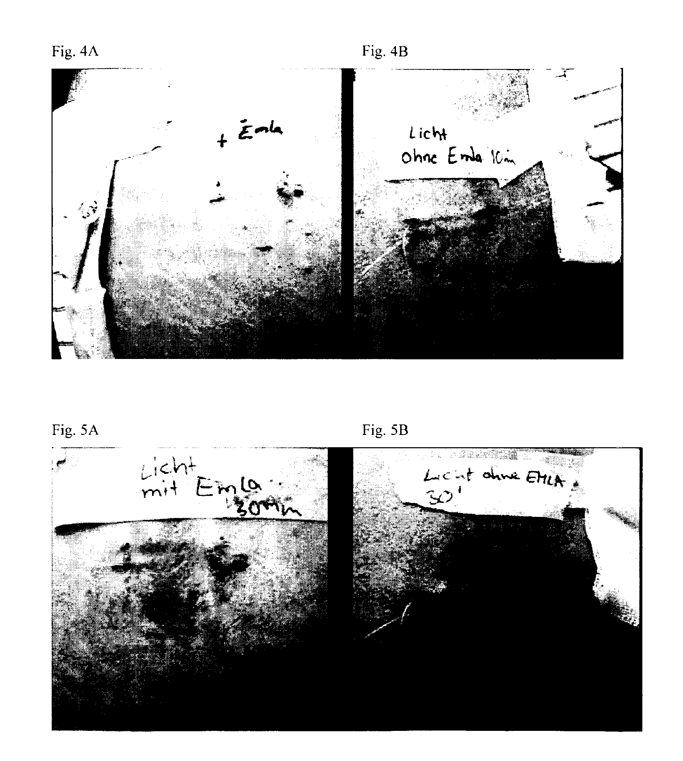

Figure 4 shows the buttock of a patient directly after the end of UVA-provocation with (Figure 4A) and without (Figure 4B) prior application of EMLA^{®}. No symptoms could be observed.
Figure 5 shows the same experiment as in Figure 4, 30 min after UV-provocation. The clinical symptoms (wheal and erythema size) were much more pronounced in the non-EMLA^{®} skin site (Figure 5B) compared to the anaesthesized site (Figure 5A).

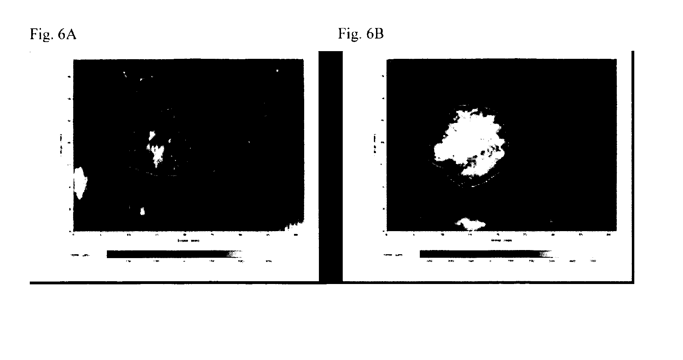

Figure 6 shows the results of digital time lapse photography of wheal formation in buttocks of a patient directly after the end of UVA provocation with (Figure 6A) and without (Figure 6B) prior application of EMLA^{®} 30 min after UV-provocation. The kinetics of skin lesion development after 30 min was documented and the results showed that wheals of the skin without prior application of EMLA^{®} covered a larger area than the ones on the pre-treated skin area.

### EXAMPLE 1

### 1.1. Subjects

Subjects to this experiment were two solar urticaria patients, one male (aged 29) and one female (aged 36). Both patients were sensitive to UVA-radiation (Figure 1).

### 1.2. Pharmaceutical preparation

EMLA^{®} is a topical anaesthetic cream (a eutectic mixture of lidocaine and prilocaine) widely used for superficial anaesthesia of the skin before taking blood samples or prior to superficial skin surgery. 1 g of EMLA^{®} cream (Astra Zeneca) contains 25 mg lidocaine (i.e. 2.5% by weight) and 25 mg prilocaine (i.e. 2.5% by weight).

### 1.3. Experimental protocol

The patients with solar urticaria and action spectrum to UVA underwent skin microdialysis after UVA provocation.

Microdialysis is a well-established technique for the continuous sampling of small, watersoluble molecules within the extracellular fluid space *in vivo.* It has the advantage over other sampling techniques in that it can be used in intact tissues to follow temporal variations in the generation and the release of substances at discrete locations within the tissue space.

EMLA^{®} cream (5 g per site) was applied to the skin of the volar forearm or buttocks of the two solar urticaria patients for 1.5 hours to achieve superficial anaesthesia of the skin prior to dermal insertion of microdialysis catheters (Figure 2).

Four microdialysis catheters per subject were inserted into the volar forearm or buttocks of the patient and perfused with saline. Following localised UVA provocation (provocation time was always 3.35 min) on the arm or buttocks the dialysate was collected for 30 min.

### 1.4. Results

Urticaria-like skin lesions usually develop after 5-20 min. This is demonstrated in Figure 1, displaying a dose-dependent whealing reaction on the buttocks of both patients after UVA radiation. Both patients showed a similar but slightly weaker reaction to UVA light on their volar forearms (photos not shown). The lesser symptoms may be explained by tolerance induction of skin sites such as the forearms which are more frequently exposed to the sun as compared to the buttocks.

In the previously anaesthesized skin (as prepared for microdialysis) of the volar forearm, however, neither wheals, nor erythema or pruritus were seen 20-30 min after UVA provocation in both patients (Figure 3).

### EXAMPLE 2

For comparison study, UV-provocation (and microdialysis) was performed a second time in skin without previous sun exposure, namely the buttocks. Thus, the female patient of Example 1 underwent the same procedure as described in Example 1 for the volar forearm again on the buttocks. A control area was irradiated with UVA but without prior application of EMLA^{®} (Figure 4).

Fifteen min after UVA radiation an erythema developed in both skin sites previously treated with and without EMLA^{®}. A whealing reaction was seen after 15 min in the non-EMLA^{®} skin site and after 20 min in the EMLA^{®}-treated skin area, but less pronounced. After 30 min the clinical symptoms (wheal and erythema size) were much more pronounced in the non-EMLA^{®} skin site compared to the anaesthesized site (Figure 5). The kinetics of skin lesion development after 30 min was also documented by digital time lapse photography (Figure 6).

### References

Güzelbey O., Ardelean E., Magerl M., Zuberbier T., Maurer M., Metz M. (2008) "Successful treatment of solar urticaria with anti-immunoglobulin E therapy", Allergy 63: 1559-1565.
Zuberbier T., Bindslev-Jensen C., Canonica W., Grattan C. E. H., Greaves M. W., Henz B. M., Kapp A., Kozel M. M. A., Maurer M., Merk H. F., Schäfer T., Simon D., Vena G. A., Wedi B. (2006) "EAACI/GA2LEN/EDF guideline: definition, classification and diagnosis of urticaria" Allergy 61: 316-320.

## Claims

1. A local anesthetic for use in the treatment or prevention of physical urticaria.

2. The local anesthetic according to claim 1, wherein the local anesthetic is selected from the group of aminoamide local anesthetics.

3. The local anesthetic according to claim 1 or 2, wherein the local anesthetic is lidocaine, or a derivative thereof, or is prilocaine, or a derivative thereof.

4. A combination of lidocaine, or a derivative thereof, and prilocaine, or a derivative thereof, for use in the treatment or prevention of physical urticaria.

5. A pharmaceutical composition comprising at least one local anesthetic for use in the treatment or prevention of physical urticaria.

6. The pharmaceutical composition according to claim 5, wherein at least one local anesthetic is selected from the group of aminoamide local anesthetics.

7. The pharmaceutical composition according to claim 5 or 6, wherein at least one local anesthetic is lidocaine, or a derivative thereof, or is prilocaine, or a derivative thereof.

8. The pharmaceutical composition according to any of claims 5 to 7, wherein the pharmaceutical composition comprises both lidocaine, or a derivative thereof, and prilocaine, or a derivative thereof.

9. The pharmaceutical composition according to any of claims 5 to 8, wherein the pharmaceutical composition is comprised by a pharmaceutical dosage form selected from the group consisting of a cream, an ointment, a salve, an elixier, a lotion, a solution, a patch, an emulsion, a foam, and a spray.

10. The pharmaceutical composition according to any of claims 5 to 9, wherein the pharmaceutical composition is a cream comprising 2.5% by weight lidocaine and 2.5% by weight prilocaine.

11. The local anesthetic according to any of claims 1 to 3, or the combination according to claim 4, or the pharmaceutical composition according to any of claims 5 to 10, wherein the physical urticaria is selected from the group consisting of cold contact urticaria, delayed pressure urticaria, heat contact urticaria, urticaria factitia/dermographic urticaria, vibratory urticaria/angioedema, and solar urticaria.

12. The local anesthetic according to claim 11, or the combination according to claim 11, or the pharmaceutical composition according to claim 11, wherein the physical urticaria is solar urticaria.
